(19) Europäisches Patentamt
European Patent Office
Office européen des brevets

(11) **EP 1 813 250 A1**

(12) **EUROPEAN PATENT APPLICATION**
published in accordance with Art. 158(3) EPC

(43) Date of publication:
**01.08.2007 Bulletin 2007/31**

(51) Int Cl.:
***A61K 6/00*** *(2006.01)*

(21) Application number: **05793790.6**

(22) Date of filing: **05.10.2005**

(86) International application number:
**PCT/JP2005/018742**

(87) International publication number:
**WO 2006/038721 (13.04.2006 Gazette 2006/15)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **07.10.2004 JP 2004294905**

(71) Applicant: **Toyo Shinyaku Co., Ltd.**
**Fukuoka-shi, Fukuoka 812-0011 (JP)**

(72) Inventor: **TAKAGAKI, Kinya,**
**c/o Toyo Shinyaku Co., Ltd.**
**Fukuoka-shi, Fukuoka 812-0011 (JP)**

(74) Representative: **Koepe, Gerd L.**
**Koepe & Partner**
**Robert-Koch-Strasse 1**
**80538 München (DE)**

(54) **EXTERNAL PREPARATION**

(57) The present invention provides an external preparation that contains processed pueraria flower. When the external preparation of the present invention is applied to skin or mucous membranes, then skin quality improving effects (skin beautifying effects) such as preventing chapped skin or preventing wrinkles, the effect of strengthening mucous membrane elasticity, and the effect of preventing gingivitis and periodontal disease, for example, are obtained. The external preparation of the present invention can develop new use of the pueraria flower.

**Description**

Technical Field

The present invention relates to external preparations.

Background Art

Kudzu, which is a leguminous plant, has been used as a raw material for food, a raw material of Chinese herbal medicines and the like for a long time. For example, the starch from kudzu has been used as a raw material of traditional Japanese confectionery. The root and flower of kudzu, which are respectively called pueraria root and pueraria flower, have been used as raw materials of Chinese herbal medicines for antipyretic, analgesic and spasmolytic effects, and improvement of symptoms such as sweating.

Among these, pueraria flower is known to contain various components, including flavonoids. In recent years, it has become clear that pueraria flower has an effect of improving hepatopathy, an effect of preventing a hangover, an effect of improving urinary nitrogen metabolism, and other effects, which are not provided by other leguminous plants (e.g., Japanese Patent No. 3454718, Japanese Patent Publication No. 08-032632, and Japanese Laid-Open Patent Publication No. 64-68318). However, only the functions of pueraria flower when taken orally in the form of Chinese herbal medicines or food have been investigated, and there has not been sufficient elucidation of its effects when administered percutaneously, for example. Thus, a new use (external preparations, etc.) of pueraria flower is desired.

Disclosure of the Invention

The inventors conducted research with an object to find a novel function of the pueraria flower and develop new use of pueraria flower. As a result, the inventors found that when a processed pueraria flower is applied to skin or mucous membranes, beneficial effects such inhibition of collagenase in skin and in mucous membranes of the oral cavity can be obtained, and thereby the present invention was achieved.

The external preparation of the present invention comprises a processed pueraria flower. This external preparation is applied to skin or mucous membranes, and exhibits the following effects: a skin quality improving effect (i.e., skin beautifying effect) on skin conditions such as chapped skin, wrinkles, and drooping; the effect of improving blemishes and increasing metabolism due to the cell activation; the effect of enhancing the elasticity of mucous membranes such as those in the oral cavity; and the effect of preventing gingivitis and periodontal disease. These effects can be obtained by the processed pueraria flower, which has beneficial effects on skin and mucous membranes, such as collagenase inhibiting effect, antioxidant effect, and collagen synthesis promoting effect. The processed pueraria flower that is contained in the external preparation of the present invention in particular has a collagenase inhibiting effect, and the effect is superior to that of other processed plant products such as soybean extract.

Best Mode for Carrying Out the Invention

The invention is described in detail below.

Processed Pueraria Flower

There are no limitations regarding the processed pueraria flower that is used in the external preparation of the present invention, so long as it is obtained by processing any flower of a pueraria plant, which belongs to the family Leguminosae. The pueraria flower includes flowers collected at various stages from flower bud to fully opened flower. In the present invention, it is particularly preferable to use buds. "A processed pueraria flower" as used in the specification refers to a product obtained by subjecting the pueraria flower to at least one of crushing, drying, pulverizing, and extraction treatments. The processed pueraria flower includes crushed pueraria flower (i.e., including the juice of the pueraria flower), dried pueraria flower, dried and pulverized pueraria flower (pueraria flower powder), and pueraria flower extract. The pueraria flower extract includes extracts that are obtained by extraction from pueraria flower, crushed pueraria flower, dried pueraria flower, or pueraria flower powder. There are no limitations regarding the form of the pueraria flower extract. The extract may be in the form of liquid, paste, or powder. In the case of powder, it may be said to pueraria flower extract powder or extract powder. Preferably it is the juice of the pueraria flower, pueraria flower extract, or dried powders thereof (i.e., juice powder, extract powder).

The processed pueraria flower used in the present invention contains flavonoids such as isoflavones, saponin, tryptophan glycoside and the like and can preferably contain isoflavones and saponin. There are no particular limitations on the contents of these components. In general, isoflavones are contained at 0.5 wt% or more, preferably 1 wt% to 20 wt% in terms of dry weight in the processed pueraria flower. Saponin is contained at 0.2 wt% or more, preferably 0.5 wt% to 20 wt%. Among the processed pueraria flowers, those processed pueraria flowers such as the juice of the pueraria flower, pueraria flower extract, or dried powders of the juice or the extract from which insoluble solids (i.e., residue) have been removed in advance, may be preferably used as a processed pueraria flower containing isoflavone and saponin abundantly. Such the processed pueraria flowers from which insoluble solids have been removed preferably contain, by dry weight, isoflavones at 3 wt% or more and more preferably 5 wt% to 90 wt%, and preferably contain saponins at 1 wt% or more and more preferably 2 wt% to 50 wt%.

The processed pueraria flower used in the present invention exhibits various excellent effects when applied to skin or mucous membranes, such as a collagenase inhibiting effect, an antioxidant effect, and a collagen synthesis-promoting effect. In particular, the collagenase inhibiting effect is greater than that of a case where a processed plant product such as a soybean extract or pueraria root extract is applied, wherein the plant product contains isoflavones or saponins in an equivalent amount with the processed pueraria flower. The processed pueraria flower with such beneficial activity for the skin or mucous membranes can exhibit the following effects; the skin quality improving effect (i.e., skin beautifying effect) such as preventing chapped skin and wrinkles; the effect of enhancing the elasticity of mucous membranes; and the effect of preventing gingivitis and periodontal disease.

Hereinafter, methods for preparing the dried pueraria flower, pueraria flower powder, and pueraria flower extract, which are the above-described processed pueraria flower, will be described.

The dried pueraria flower is obtained by drying pueraria flower, preferably in the bud stage, by any methods such as drying in the sun or hot air drying. Preferably, such a drying is performed until the moisture content becomes 10 wt% or less.

The pueraria flower powder can be obtained by pulverizing the above-described dried pueraria flower. Pulverization is performed by any methods commonly used by those skilled in the art, for example, by using a ball mill, a hammer mill, a roller mill or the like.

The pueraria flower powder can also be obtained by crushing the collected raw pueraria flower using a masscolloider, a slicer, a comitrol or the like to give a crushed pueraria flower and drying the crushed pueraria flower.

The pueraria flower extract can be obtained by, for example, adding a solvent to collected raw pueraria flower, crushed pueraria flower, dried pueraria flower, or pueraria flower powder (hereinafter they are sometimes collectively referred to simply as a raw material for extraction) and optionally heating them to give an extract, and collecting the extract by centrifugation or filtration.

Examples of the solvent used in the above-described extraction include water, an organic solvent, and an aqueous organic solvent. Examples of the organic solvent include methanol, ethanol, n-propanol, n-butanol, acetone, hexane, cyclohexane, propylene glycol, methyl ethyl ketone, glycerin, methyl acetate, ethyl acetate, diethyl ether, dichloromethane, edible oils and fats, 1,1,1,2-tetrafluoroethane, and 1,1,2-trichloroethene. A polar organic solvent is preferable, ethanol, n-butanol, methanol, acetone, propylene glycol, and ethyl acetate are more preferable, and ethanol is particularly preferable.

There is no particular limitation on the extraction temperature, as long as the extraction temperature is not more than the boiling point of the solvent to be used. Although the extraction temperature varies depending on the solvent to be used, it is generally 4°C to 130°C in view of the degradation of an active component, for example. Preferably, the extraction temperature is 50°C to 130°C, more preferably 70°C to 100°C. When heating is performed for extraction, for example, heat extraction such as heating under reflux and supercritical fluid extraction can be employed. When heating is performed, heating may be performed under pressure.

The extraction time can be any length of time, for which a soluble component is extracted from the raw material for extraction in a sufficient amount, and the extraction time can be established as appropriate according to the extraction temperature and the like. Preferably, the extraction time is 30 minutes to 48 hours. For example, the extraction time may be 6 hours to 48 hours at a temperature below 50°C. The extraction time may be performed for 30 minutes to 24 hours at a temperature of 50°C or above.

The pueraria flower extract can be optionally concentrated by any methods used by those skilled in the art, such as concentration under reduced pressure and freeze-drying, to give a liquid, paste or powder form of the extract.

Alternatively, the pueraria flower extract can be purified using a synthetic adsorbent (DIAION HP20, SEPABEADS SP825, Amberlite XAD4, MCIgelCHP20P, etc.) or a dextran resin (Sephadex LH-20, etc.), to enhance the concentrations of flavonoid, saponin and the like.

External Preparation

The external preparation of the present invention contains processed pueraria flower. There are no particular limitations regarding the content of processed pueraria flower, however the content can be adjusted as appropriate according to the form of the processed pueraria flower. The processed pueraria flower is contained at preferably 0.00001 to 40 wt%, and more preferably 0.001 to 20 wt% in terms of dry weight in the external preparation.

The external preparation of the present invention may also include auxiliary agent such as various types of medicinal components, oil, humectant, surfactant, and ultraviolet absorber, for the purpose of enhancing or augmenting the activity of the processed pueraria flower. The auxiliary agent may be used alone or in combination of two or more.

Examples of the medicinal components, which are one of auxiliary agents, include active oxygen scavenger; vitamins, vitamin-like active substance and salts or derivatives of these; polyphenol; cell activator; antiinflammatory agent; phospholipid; hormone agents; and extract derived from plants or animals having the effects of these.

Examples of active oxygen scavenger and antioxidant include carotenoids, L-cysteine and derivatives and salts thereof, SOD, mannitol, hydroquinone, tryptophan, histidine, flavonoids (quercetine, hesperidin, etc.), gallic acid and derivatives thereof, BHT, BHA, and plant extracts containing these components (moutan cortex extract, tomato extract, parsley extract, Melissa extract, scutellaria root extract, etc.).

Examples of vitamins include vitamin A such as retinol, retinal, retinoic acid, 3-dehydroretinol, 3-dehydroretinal, and 3-dehydroretinoic acid; provitamin A such as $\alpha$-carotene, $\beta$-carotene, $\gamma$-carotene, and cryptoxanthin; vitamin B family such as thiamine (vitamin $B_1$), riboflavin (vitamin $B_2$), pyridoxine, pyridoxal, and pyridoxamine (these are vitamin $B_6$), cobalamine (vitamin $B_{12}$), nicotinic acid, nicotinamide, pantothenic acid, biotin (vitamin H), and folic acid (vitamin M); ascorbic acid (vitamin C); vitamin D such as ergocalciferol (vitamin $D_2$) and cholecalciferol (vitamin $D_3$); provitamin D such as 7-dehydrochloestrol and ergosterol; vitamin E such as $\alpha$-tocopherol, $\beta$-tocopherol, $\gamma$-tocopherol, $\delta$-tocopherol, $\alpha$-tocotrienol, $\beta$-tocotrienol, $\gamma$-tocotrienol, and $\delta$-tocotrienol; and vitamin K such as phylloquinone (vitamin $K_1$), menaquinone (vitamin $K_2$), and menadione (vitamin $K_3$).

Examples of vitamin-like active substance includes ubiquinone, lipoic acid (thioctic acid), essential fatty acid (vitamin F) (such as linoleic acid, linolenic acid, arachidonic acid), orotic acid (vitamin $B_{13}$), carnitine (vitamin $B_T$), myo-inositol, choline, vitamin P (such as rutin, hesperidin, and eriocitrin), methyl-methionine-sulfonium (vitamin U), and pantothenyl alcohol.

The vitamins and the vitamin-like active substance may be used without limiting to optical isomers, stereoisomers, or the like. For example, it is also possible to use a mixture of cis and trans forms or racemic substance thereof.

Examples of salts of the vitamins and the vitamin-like active substance include alkali metal salts such as sodium salts and potassium salts; alkaline earth metal salts such as magnesium salts and calcium salts; inorganic acid salts such as hydrochlorides and sulfates; organic acid salts such as ammonium salts, lactates, and acetates; and amine salts such as triethanolamine salts.

Examples of derivatives of the vitamins and the vitamin-like active substance include alkyl esters, alkenyl esters, aryl esters, phosphate esters, sulfate esters, phosphatidyl esters, and glycosides (e.g., glucosides, galactosides, maltosides, and lactosides) of the vitamins or the vitamin-like active substance.

As for the vitamins, the vitamin-like active substance, and the salts and derivates thereof, both those obtained from natural sources such as plants and animals, algae, and microorganisms, and those obtained synthetically by chemical reactions or enzymatic reactions, may be used. Additionally, the extract of natural products containing these may be used as is.

Examples of polyphenol includes catechol, catechin, epicatechin, gallocatechin, epigallocatechin, gallocatechin gallate, epigallocatechin gallate, tannic acid (m-galloyl gallic acid), hamamelitannin (1,5-digalloyl hamamelose), proanthocyanidin, caffeic acid derivatives, gallic acid, pyrogallol tannin, and catechol tannin. As the polyphenol, it is also possible to use tea extract, grape seed extract, pine bark extract, and sweet potato stalk and leaf extract, for example, containing these polyphenols.

Examples of cell activator includes the following materials: extract or culture supernatant of yeast; the extracts of Asparagus plants, Persea americana Mill., Aloe plants, Prunus armeniaca L. var. ansu Maxim., Ginkgo biloba L., Fagus japonica Maxim., Allium sativum L. f. pekinense Makino, Panax ginseng C.A. Meyer, Matricaria chamomilla L., Phellodendron amurense Rupr. and Phellodendron plants, Cucumis sativus L., Calendula arvensis L., Lentinus edodes Sing., Actinidia chinensis Planch. (kiwi), Equisetum arvense L., Aesculus hippocastanum L., Allium sativum L., Swertia japonica Makino, Stephania cepharantha Hayata, Lactuca sativa L. (lettuce), Capsicum annuum L., Calendula officinalis L., Aesculus turbinata Blume, Daucus carota L., Poria cocos Wolf (pachyma hoelon), Vitis vinifera, L., Fagus crenata Blume, Luffa cylindrica M. Roemen, Carthamus tinctorius L., Rosmarinus officinalis L., Citrus plants, Sapindus mukurossi Gaertn., Lithospermum officinale L. var. erythrorhizon Maxim., Eucalyptus plants, and Lilium plants; hydroxy fatty acids and the salts and derivatives thereof; nucleic acids and nucleic acid-related substances (for example, deoxyribonucleic acid sodium salt and deoxyribonucleic acid potassium salt); and mixtures of proteins extracted from egg shell membrane and isomerized sugar. The cell activator may be used alone or in combination of two or more.

Examples of antiinflammatory agent includes the following compounds and materials: steroidal antiinflammatory agent (such as cortisone, hydrocortisone, prednisolone, methylprednisolone, dexamethasone, betamethasone, triamcinolone, triamcinolone acetonide, fluocinolone acetonide, fluocinonide, beclometasone, and phosphates, propionates, acetates, and succinates thereof); non-steroidal antiinflammatory agent (for example, salicylic acid and derivatives thereof (such as aspirin, salicylamide, ethenzamide, and methyl salicylate), indole acetic acid derivatives (such as indomethacin and sulindac), pyrazolidinedione derivatives (such as phenylbutazone and oxyphenbutazone), anthranil derivatives (such as mefenamic acid and flufenamic acid), propionic acid derivatives (such as ibuprofen, ketoprofen, and naproxen), phenyl acetic acid derivatives (such diclofenac, fenbufen and bufexamac), and benzothiazine derivatives (such as piroxicam)); glycyrrhizinic acid and salts and derivatives thereof (such as dipotassium glycyrrhizinate and monoammonium glycyrrhizinate); glycyrrhetinic acid and salts and derivatives thereof (such as stearyl glycyrrhetinate, glycyrrhetinyl stearate, and disodium 3-succinyloxy glycyrrhetinate); azulene derivatives (such as guaiazulene, ethyl guaiazulene sulfonate, sodium guaiazulene sulfonate, and chamazulene); allantoin; aloin; aloe emodin; shikonin and derivatives thereof (such as isobutylshikonin, acetylshikonin, and isovalerylshikonin); ginsenoside (such as ginsenoside $R_{a1}$, ginsenoside $R_{a2}$, and ginsenoside $R_{b1}$) and derivatives thereof (20-glucoginsenoside $R_f$); paeoniflorin; and paeonol and derivatives thereof (such as paeonoside and paeonolide). Plant extracts containing various antiinflammatory agents can be used as the antiinflammatory agent. Examples of the plant extracts include the extracts from Scutellariae Radix, Glycyrrhizae Radix, Sophorae Radix, Bupleuri Radix, Paeoniae Radix, Cimicifugae Rhizoma, Zizyphi Fructus, Anemarrhenae Rhizoma, Moutan Cortex, Gentianae Scabrae Radix, and Forsythiae Fructus.

Phospholipid can be contained for the purpose of a moisturizing effect and a cell activating effect. Examples of phospholipid includes glycerophospholipid such as phosphatidylcholine, phosphatidylethanolamine, phosphatidylserine, phosphatidylinositol, phosphatidylglycerol, diphosphatidylglycerol (cardiolipin), and phosphatidic acid; lysoglycerophospholipid such as lysophosphatidylcholine (lysolecithin), lysophosphatidylethanolamine, lysophosphatidylserine, lysophosphatidylinositol, lysophosphatidylglycerol, and lysophosphatidic acid; sphingophospholipid such as sphingomyelin; and hydrogenates of these. The phospholipid can be derived from plants or animals such as soybean and egg yolk, or can be synthesized by chemical or enzymatic methods.

Examples of hormone agent include estradiol or derivatives thereof; and plant hormone-like substance (isoflavone such as genistein and daidzein).

Examples of oil, which is one of the auxiliary agent, includes unsaturated fatty acid such as linoleic acid, linolenic acid, palmitic acid, DHA, and EPA, and derivatives thereof, and oils extracted from plants and animals such as linseed oil, coconut oil, jojoba oil, olive oil, squalane, squalene, horse oil, rice bran oil, and castor oil, and derivatives thereof.

Examples of humectant, which is one of the auxiliary agent, includes mucopolysaccharide such as collagen and the decomposition product thereof, collagen-like peptides that are included in carrot extract, for example, soybean peptide, amino acid, and hyaluronic acid; amino sugar such as chondroitin; sugar such as trehalose; and water soluble dietary fiber such as seaweeds, alginic acid, glucomannan, and pectin.

Examples of surfactant, which is one of the auxiliary agent, includes lecithin, fatty acid ester, and amino acid derivatives.

Examples of ultraviolet absorber, which is one of the auxiliary agent, includes zinc oxide and titanium oxide.

There are no particular limitations regarding the form of the external preparation of the present invention. It can take the form of lotion, emulsion, gel, cream, ointment, powder, or granule, for example. Depending on its form, for the purpose of increasing the outward appearance, the feeling of use, and the storage stability, the external preparation may also suitably contain excipient such as dextrin; perfume; pigment; preservative; thickener such as acrylic polymer and carboxyvinyl polymer; chelating agent such as EDTA; sweetening agent such as sucralose; refrigerant such as menthol; antiseptic/antifungal agent such as phenoxyethanol; and base such as glycerol, ethanol, paraben, and butylene glycol.

The external preparation of the present invention may be employed as a drug, a quasi-drug, a cosmetic, and a toiletry item, for example. Specifically, it can be employed as a skin lotion, a facial cream, a milky lotion, a cream, a pack, a hair tonic, a hair cream, a shampoo, a conditioner, a hair treatment, a body shampoo, a facial cleanser, a soap, a foundation, a face powder, a lipstick, a lip gloss, a rouge, an eye shadow, a hairdressing, a hair restorer, a water based ointment, an oil-based ointment, an eye drop, an eye wash, a fomentation, and a gel. If it is to be applied to the inside of the oral cavity, then it may also serve as a dentifrice, mouthwash, spray, gum, hard candy, tablet, or gummi, for example.

When the external preparation of the present invention is applied to skin or mucous membranes, such as when it is applied or sprayed, the external preparation achieves the following excellent effects: a skin quality improving effect (skin beautifying effect) on skin conditions such as chapped skin, wrinkles, and drooping; the effect of improving blemishes and increasing metabolism due to the cell activation; the effect of enhancing the elasticity of the mucous membranes such as those in the oral cavity; and the effect of preventing gingivitis and periodontal disease. These effects can be obtained from processed pueraria flower, which has beneficial effects for skin and mucous membranes, such as collagenase inhibiting effect, antioxidant effect, and collagen synthesis-promoting effect.

Examples

The present invention is described below through examples. These examples are not intended to limit the present invention.

Example 1: Collagenase inhibiting activity

(1) Preparing a test solution

A test solution was prepared by adding pueraria flower extract (containing isoflavones at 10 wt% and saponins at 3 wt%; manufactured by Ohta's Isan Co., Ltd.) to a 0.05 M of tricine buffer solution (pH 7.5) to a concentration of 10 μL/mL.

(2) Preparing an enzyme solution

An enzyme solution of a collagenase B (Roche Diagnostics GmbH) was prepared by diluting the enzyme with 0.05 M of tricine buffer solution (pH 7.5) to a concentration of 0.14 U/mL.

(3) Preparing a substrate solution

A substrate solution was prepared by dissolving 5 mg of N-(3-[2-furyl]acryloyl)-Leu-Gly-Pro-Ala (manufactured by Sigma Chemical) in 35 mL of 0.05 M of tricine buffer solution (pH 7.5).

(4) Measuring the collagenase inhibiting activity

First, 100 μL of test solution and 100 μL of enzyme solution were mixed and incubated for one hour at 37°C. Then, 100 μL of substrate solution was added to this mixture, and this was further incubated for three minutes at 37°C. Then, the absorption of this mixture at 324 nm was measured (this shall be a measured value A). The absorption of the blank was measured in the same procedure as described above, except that 0.05 M of tricine buffer solution (pH 7.5) was used instead of the enzyme solution (this shall be a measured value a). On the other hand, as a control, the absorption of the 0.05 M tricine buffer solution (pH 7.5) (this shall be a measured value B) and the absorption of the blank (this shall be a measured value b) were measured in the same procedure as described above, except that the 0.05 M tricine buffer solution (pH 7.5) was used instead of the test solution. It should be noted that each measurement was performed in triple measurements. The measured values that were obtained were used to calculate the collagenase inhibition rate (%) from the following formula. The results are shown in Table 1.

$$\text{collagenase inhibition rate (\%)} = \{1-(A-a)/(B-b)\} \times 100$$

Comparative Example 1

A test solution was prepared in the same manner as in Example 1, except that a soybean extract (containing isoflavones at 10 wt%; manufactured by Fuji Oil Co., Ltd.) was used instead of a pueraria flower extract, and the collagenase inhibiting activity was measured. The results are shown in Table 1.

Table 1

|  | Extract | Collagenase inhibition rate (%) |
|---|---|---|
| Ex. 1 | Pueraria flower extract | $52.4 \pm 14.0$ |
| Com. Ex. | Soybean extract | $18.7 \pm 9.7$ |
| Each of the values indicates mean value $\pm$ standard deviation. | | |

From the results of Table 1, it can be understood that the pueraria flower extract has an excellent collagenase inhibiting effect (Example 1). The collagenase inhibiting effect that is obtained from the pueraria flower extract is significantly better than that of a soybean extract that contains the same amount of isoflavones (Comparative Example 1). This demonstrates that the processed pueraria flower that is used in the present invention has a superior collagenase inhibiting effect compared to that of isoflavones, or processed plants that contain isoflavones, which conventionally have been known as collagenase inhibiting agents. Thus, it is considered that it will exhibit the following effects: a skin quality improving effect (skin beautifying effect) on skin conditions such as chapped skin, wrinkles, and drooping; the effect of improving blemishes and increasing metabolism due to the cell activation; the effect of enhancing the elasticity of mucous membranes such as those in the oral cavity; and the effect of preventing gingivitis and periodontal disease.

Example 2: Skin quality improving effect

(1) Preparing a cosmetic lotion

Using the pueraria flower extract that was used in Example 1, the following cosmetic lotion (cosmetic 1) was prepared. The mixed components and the mixing amounts are shown below.

| <Mixed components in the cosmetic lotion> | Mixing amount (wt%) |
| --- | --- |
| Pueraria flower extract | 0.002 |
| Ethanol | 10.0 |
| Polyoxyethylene hardened castor oil | 1.0 |
| Methyl parahydroxybenzoate | 0.1 |
| Dipropylene glycol | 5.0 |
| 1,3-Butylene glycol | 10.0 |
| Purified water | remainder |

Separately from the above, a cosmetic lotion was prepared in the same manner as above, except that the soybean extract that was used in Comparative Example 1 was used instead of the pueraria flower extract. This cosmetic lotion is referred to as cosmetic 2.

(2) Evaluating the skin quality improving effect

The skin quality improving effect of skin was evaluated using the cosmetics 1 and 2. First, 40 female panelists in their 30s, 40s and 50s with the subjective symptoms of the formation of fine wrinkles and a drop in skin elasticity were served as the panelists. Then, 20 panelists used cosmetic 1, and the remaining 20 panelists used cosmetic 2, the identity of the cosmetic unknown to them, applying the cosmetic to the surface of the skin on their face twice a day for one month. The panelists then filled out a questionnaire on the formation of fine wrinkles and skin elasticity, scoring these based on the following criteria. When both the total score for the formation of fine wrinkles and the total score for the skin elasticity were below 20 points, then it was determined that the skin quality improving effect is insufficient ($\times$). When either one of the total score for the formation of fine wrinkles and the total score for the skin elasticity was 20 points or more, it was determined that there is a skin quality improving effect but that it is not sufficient (open triangle). When both of the total score for the formation of fine wrinkles and the total score for the skin elasticity were 20 points or more, it was determined that there is an excellent skin quality improving effect (double circle). The results are shown in Table 2.

(Formation of fine wrinkles and skin elasticity)

2 points: Improvement compared to before using the cosmetic
1 point: Slight improvement compared to before using the cosmetic
0 point: No change from that before using cosmetic

Table 2

| | Skin quality improving effect | | | | | | | | |
| --- | --- | --- | --- | --- | --- | --- | --- | --- | --- |
| | Formation of fine wrinkles | | | | Skin elasticity | | | | Evaluation |
| | Improved (2 points) | Slightly improved (1 point) | No change (0 points) | Total points | Improved (2 points) | Slightly improved (1 point) | No change (0 points) | Total points | |
| Cosmetic 1 | 10 panelists | 6 panelists | 4 panelists | 26 points | 13 panelists | 4 panelists | 3 panelists | 30 points | ◎ |
| Cosmetic 2 | 3 panelists | 9 panelists | 8 panelists | 15 points | 7 panelists | 9 panelists | 4 panelists | 23 points | △ |

From the results of Table 2, it can be understood that cosmetic 1, which contains the pueraria flower extract, received more than 20 points both for the score indicating the improvement in fine wrinkles and the score indicating the skin elasticity, and thus has a greater skin quality improving effect than cosmetic 2, which contains soybean extract with the same amount of isoflavones.

Example 3: Preparing a composition for oral cavity

Using the pueraria flower extract that was used in Example 1, the following composition for oral cavity (100 mg per tablet) was prepared. The mixed components and the mixing amounts are shown below.

| <Mixed components in the composition for oral cavity> | Mixing amount (wt%) |
| --- | --- |
| Pueraria flower extract | 0.4 |
| Granular sugar | 57.0 |
| Lactose | 20.0 |
| Maltitol | 19.6 |
| Sucrose fatty acid ester | 2.0 |
| Silicon dioxide | 1.0 |

Example 4: Preparing a skin lotion

To a mixture having the following mixing amounts containing the pueraria flower extract that was used in Example 1, purified water was added to a total amount of 100 g, and this was stirred and mixed until uniform, producing a skin lotion.

| <Mixed components in the skin lotion> | Mixing amount (g) |
| --- | --- |
| Pueraria flower extract | 0.002 |
| Glycerin | 6 |
| Propylene glycol | 4 |
| Oleyl alcohol | 0.1 |
| Polyoxyethylene lauryl ether | 1 |
| Ethanol | 5 |
| Phenoxyethanol | 0.1 |
| Ascorbic acid | 1 |

Example 5: Preparing a W/O emollient cream

To a mixture having the following mixing amounts containing the pueraria flower extract that was used in Example 1, purified water was added to a total amount of 100 g, and this was stirred and mixed until uniform, producing a W/O emollient cream. This emollient cream exhibits a collagenase inhibiting effect in addition to a moisturizing effect and a keratolytic effect, and thus it is useful as a skin external preparation.

| <Mixed components in the W/O emollient cream> | Mixing amount (g) |
| --- | --- |
| Pueraria flower extract | 0.01 |
| Microcrystalline wax | 3 |
| Lanolin | 3 |
| Vaseline | 5 |
| Squalane | 9 |
| Olive oil | 12 |
| Sorbitan sesquioleate | 3 |
| Polyoxyethylene sorbitan trioleate (20 E.O.) | 0.5 |
| Sorbitol | 9 |
| Tocopherol | 0.01 |
| Antiseptic agent | suitable amount |
| Perfume | suitable amount |

Example 6: Evaluating the ability to suppress the generation of active oxygen caused by ultraviolet radiation

HFSKF-II cells (human skin fibroblast; obtained from RIKEN) were seeded in a 96-well plate at a concentration of $2 \times 10^5$ cells/well, and using 0.1 mL of standard medium (Ham's F-12 medium containing 15% of fetal calf serum; manufactured by Sigma-Aldrich), these were incubated for 24 hours at 37°C in a 5% $CO_2$ atmosphere. Next, the plate was washed three times with PBS by the method of Hong Wang et al. (Free Radic. Biol. Med., 1999). Then the medium was replaced with a standard medium containing 100 $\mu$M of DCFH-DA (2',7'-dichlorofluorescein diacetate; manufactured by Sigma-Aldrich) and this was incubated for 10 minutes at 37°C in a 5% $CO_2$ atmosphere. Next, the plate was washed three times with PBS. For six wells, the medium was replaced with a standard medium containing 100 $\mu$g/mL of pueraria flower extract (containing isoflavones at 10 wt% and saponin at 3 wt%; manufactured by Ohta's Isan Co., Ltd.) and 0.03 wt% of hydrogen peroxide (these serve as the test group). Next, using a handy UV lamp (LUV-4; AS ONE CORPORA-TION), UV A ($\lambda$=365 nm) was irradiated at 4.4 J/cm². UV A is a UV radiation that readily passes through skin, and is known to promote skin aging.

Next, the fluorescence F of the DCFH-DA that is emitted due to the presence of active oxygen was measured with a multispectrometer (Varioscan; excitation wavelength: 485 $\pm$ 10 nm, detection wavelength: 530 $\pm$ 10 nm; Thermo Electron Corporation), and the suppression rate of the active oxygen generation due to UV radiation when the pueraria flower extract was employed was calculated from the following formula. The results are shown in Table 3.

$$\text{Suppression rate of the active oxygen generation (\%)} = \frac{F_{control} - F_{sample}}{F_{control} - F_{blank}} \times 100$$

$F_{sample}$: The fluorescence measured after the medium was replaced with a standard medium that contains pueraria flower extract and UV A was irradiated.

$F_{control}$: The fluorescence measured after the medium was replaced with a standard medium that does not contain pueraria flower extract and UV A was irradiated.

$F_{blank}$: The fluorescence measured after replacing with a standard medium that does not contain pueraria flower extract, during which UV A was not irradiated.

As a comparative group, incubation was conducted under the same conditions as those for the test group, except that coenzyme Q10 (DAEWOONG Chemical Co., Ltd.) was used instead of the pueraria flower extract that was used by the test group. Then, the suppression rate of the active oxygen generation due to UV radiation was measured. The results are shown in Table 3.

Table 3

| | Test group | Comparative group |
|---|---|---|
| | Pueraria flower extract | Coenzyme Q10 |
| Suppression rate of the active oxygen generation (%) | 57.1±4.4 | 30.6±7.7 |

From the results of Table 3, it can be understood that the pueraria flower extract exhibits excellent effects of inhibiting active oxygen generation due to UV radiation compared with coenzyme Q10 exhibits, wherein the coenzyme Q10 has been frequently used in external preparations as an antioxidant in recent years. Consequently, it is clear that the pueraria flower extract inhibits the active oxygen generation due to UV radiation, and can suppress the aging of skin due to UV radiation.

Example 7: Preparing a bath preparation

A test bath preparation and a comparative bath preparation were prepared by mixing pueraria flower extract (containing isoflavones at 10 wt% and saponin at 3 wt%; manufactured by Ohta's Isan Co., Ltd.), sodium sulfate, sodium bicarbonate, perfume, and dextrin at the ratios listed in Table 4.

Table 4

|  | Test bath preparation (wt%) | Comparative bath preparation (wt%) |
|---|---|---|
| Pueraria flower extract | 75 | - |
| Sodium sulfate | - | 75 |
| Sodium bicarbonate | 15 | 15 |
| Perfume | 1 | 1 |
| Dextrin | 9 | 9 |

Example 8: Evaluation of the fragrance of the bath preparations

The fragrance of the test bath preparation and the comparative bath preparation that were prepared in Example 7 was evaluated as follows.

The test bath preparation (0.5 g) was dissolved in 45°C water (50 mL) to prepare a test bath water 1. Next, the comparative bath preparation (0.5 g) was dissolved in 45°C water (50 mL) to prepare a comparative bath water 1. Then, 20 panelists were made to smell the fragrance of the test bath water 1 and the comparative bath water 1, and evaluated these based on the following criteria.

<Evaluation Criteria>

The fragrance is mentally relaxing    1 point
No change mentally due to the fragrance    0 point
The fragrance is unpleasant    -1 point

The total score of the 20 panelists was 13 points for the test bath water 1 and 9 points for the comparative bath water 1. Thus, it was clear that the test bath preparation that contains pueraria flower extract tends to have a fragrance that brings about a mentally relaxing feeling.

Example 9: Evaluation of the bath effects of the bath preparation

The bath effect of the test bath preparation and the comparative bath preparation that were prepared in Example 7 was evaluated as follows.

The test bath preparation (10 g) was dissolved in 45°C water (1 L) to prepare a test bath water 2. Next, the comparative bath preparation (10 g) was dissolved in 45°C water (1 L) to prepare a comparative bath water 2. Next, a laser Doppler blood perfusion imager (PeriScan PIMII; made by PERIMED) was used to measure the blood flow rate of the forefinger of left hand of six panelists. The six panelists soaked (bathed) their left hand in the 45°C test bath water 2 for 10 minutes, and the blood flow rate of the forefinger of their left hand was measured immediately after soaking. Next, they soaked their left hand in 10°C cold water for 15 seconds to perform a cold water load test, and the blood flow of the forefinger of their left hand was measured one minute after the cold water load. These blood flow rates are shown in Table 5.

The same procedure was used to measure blood flow rate for the comparative bath water 2 as well. The results are shown in Table 5.

Table 5

|  | Test bath preparation | Comparative bath preparation |
|---|---|---|
| Before soaking | 1.69 | 1.67 |
| Immediately after soaking | 2.43 | 2.30 |
| One minute after the cold water load | 2.23 | 2.08 |

Each of the values indicates mean value and unit indicates volt (V).

From the results of Table 5, it was observed that the test bath preparation (test bath water 2) that contains

pueraria flower extract tends to have a greater blood flow improving effect than the comparative bath preparation (comparative bath water 2). Moreover, there was good circulation after the cold water load as well, and thus it is clear that the test bath preparation can be expected to have a blood flow improving effect. Consequently, it is clear that bath preparation that contains pueraria flower extract exhibits an excellent effect.

Industrial Applicability

When the external preparation of the present invention is applied to skin or mucous membranes, it exhibits the following excellent effects: a skin quality improving effect (skin beautifying effect) on skin conditions such as chapped skin, wrinkles, and drooping; the effect of improving blemishes and increasing metabolism due to a cell activation; the effect of enhancing the elasticity of mucous membranes such as those in the oral cavity; and the effect of preventing gingivitis and periodontal disease. The processed pueraria flower that is contained in the external preparation of the present invention in particular has an excellent collagenase inhibiting effect, and the effect is superior to that of other processed plant products such as soybean extract. The external preparation of the present invention may be employed as a drug, a quasi-drug, a cosmetic, and a toiletry item, for example.

**Claims**

1. An external preparation comprising a processed pueraria flower.

2. The external preparation of claim 1, wherein the processed pueraria flower is at least one selected from the group consisting of dried pueraria flower, pueraria flower extract, crushed pueraria flower, and dried and pulverized pueraria flower.

3. The external preparation of claim 1 or 2, wherein the external preparation has the form of a lotion, an emulsion, a gel, a cream, an ointment, a powder, or granules.

## INTERNATIONAL SEARCH REPORT

| | International application No. |
|---|---|
| | PCT/JP2005/018742 |

| A. CLASSIFICATION OF SUBJECT MATTER |
|---|
| *A61K36/48*(2006.01), *A61K8/00*(2006.01), *A61Q19/00*(2006.01), *A61P1/02* (2006.01), *A61P17/16*(2006.01), *A61P43/00*(2006.01), *A61K8/97*(2006.01), *A61P17/18*(2006.01) |
| According to International Patent Classification (IPC) or to both national classification and IPC |

| B. FIELDS SEARCHED |
|---|
| Minimum documentation searched (classification system followed by classification symbols) |
| *A61K36/48*(2006.01), *A61K8/00*(2006.01), *A61Q19/00*(2006.01), *A61P1/02* (2006.01), *A61P17/16*(2006.01), *A61P43/00*(2006.01), *A61K8/97*(2006.01), *A61P17/18*(2006.01) |
| Documentation searched other than minimum documentation to the extent that such documents are included in the fields searched |
| |
| Electronic data base consulted during the international search (name of data base and, where practicable, search terms used) |
| |

C. DOCUMENTS CONSIDERED TO BE RELEVANT

| Category* | Citation of document, with indication, where appropriate, of the relevant passages | Relevant to claim No. |
|---|---|---|
| Y | JP 1-68318 A (Ohta's Isan Co., Ltd.), 14 March, 1989 (14.03.89), (Family: none) | 1-3 |
| Y | JP 11-511471 A (Duthinh, Phu), 05 October, 1999 (05.10.99), Page 7, the last line to page 8, line 1 & US 5547671 A & EP 851761 A1 & WO 97/10835 A1 & AU 6391296 A | 1-3 |
| Y | JP 2001-39849 A (Shiseido Co., Ltd.), 13 February, 2001 (13.02.01), (Family: none) | 1-3 |
| Y | JP 2004-67590 A (Naris Cosmetics Co., Ltd.), 04 March, 2004 (04.03.04), (Family: none) | 1-3 |

| ☐ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 12 December, 2005 (12.12.05) | 27 December, 2005 (27.12.05) |

| Name and mailing address of the ISA/ | Authorized officer |
|---|---|
| Japanese Patent Office | |
| Facsimile No. | Telephone No. |

Form PCT/ISA/210 (second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3454718 B
- JP 8032632 A
- JP 6468318 A